# EUROPÄISCHE PATENTSCHRIFT

(11) **EP 3 727 540 B1**
(45) Veröffentlichungstag und Bekanntmachung des Hinweises auf die Patenterteilung: **29.12.2021**
(21) Anmeldenummer: 18839591.7
(22) Anmeldetag: 12.12.2018
(51) Int. Cl.: A61M 16/04, A61M 16/08, F16L 27/04

(54) **TRACHEALKANÜLE UND VERFAHREN ZUR HERSTELLUNG**
TRACHEAL CANNULA AND METHOD FOR PRODUCING SAME
CANULE TRACHÉALE ET PROCÉDÉ POUR SA PRODUCTION

(30) Priorität: 20.12.2017 DE 102017011803
(43) Veröffentlichungstag der Anmeldung: 28.10.2020
(73) Patentinhaber: Primed Halberstadt Medizintechnik GmbH, 38820 Halberstadt (DE)
(72) Erfinder: LEIBITZKI, Sascha, 38889 Blankenburg (DE); RAMDOHR, Bastian, 06493 Ballenstedt (DE)
(74) Vertreter: Fischer, Volker
(86) Internationale Anmeldenummer: PCT/DE2018/000368
(87) Internationale Veröffentlichungsnummer: WO 2019/120349

(56) Entgegenhaltungen:
- WO-A1-2015/162396
- CH-A- 138 387
- DE-A1-102004 002 125
- DE-A1-102012 004 359
- US-A- 3 824 999

## Beschreibung

Die Erfindung betrifft eine Trachealkanüle, welche mindestens ein gebogenes Rohr und einen Konnektor aufweist.

Die Erfindung betrifft auch ein Verfahren zur Herstellung einer Trachealkanüle, bei welchem ein gebogenes Rohr mit einem Konnektor verbunden wird.

Beispielsweise bei der Notwendigkeit einer Langzeitbeatmung nach einem Unfall oder einer Operation, einer neurologischen Erkrankung mit einer Störung des Schluckreflexes, einer Strahlenbehandlung am Kopf oder Hals oder bei einer Kehlkopflähmung kann ein chirurgischer Eingriff notwendig sein, bei welchem durch die Halsweichteile ein Zugang zur Luftröhre, welcher auch als Tracheostoma bezeichnet wird, geschaffen wird. Dieser umgangssprachlich auch als Luftröhrenschnitt bezeichnete medizinische Eingriff wird im Stand der Technik beispielsweise auch bei einer Verlegung der oberen Atemwege als letztes Mittel durchgeführt, um den Patienten vor dem Ersticken zu bewahren. Ein Tracheostoma ist auch bei Patienten nach kompletter Entfernung des Kehlkopfes üblich.

Aus dem Stand der Technik ist ebenfalls bekannt, eine sogenannte Trachealkanüle durch ein Tracheostoma in die Luftröhre einzuschieben und zu befestigen, wobei das Tracheostoma durch diese Trachealkanüle offengehalten wird. Eine derartige Trachealkanüle ist meist ein flexibler bis starrer, kurzer, gekrümmter Schlauch, durch welchen die Atmung ermöglicht oder erleichtert wird. Wird eine derartige Behandlung mit einer Trachealkanüle über einen längeren Zeitraum fortgesetzt, ist es auch üblich, die Trachealkanüle regelmäßig zu wechseln.

Trachealkanülen unterscheiden sich beispielsweise durch ihre Materialart. Für derartige Kanülen werden meist Werkstoffe wie Silikon oder andere Kunststoffe oder Metalle wie Silber bzw. Neusilber verwendet. Eine weitere Unterscheidung der Kanülen kann nach deren Länge, Innendurchmesser (Lumen), Form und Funktion erfolgen. Die Länge und das Lumen der Kanüle sind üblicherweise an die Größe des Tracheostomas des Patienten angepasst. Gemäß der Funktionsweise der Trachealkanülen kann zwischen blockbaren und nicht blockbaren Kanülen unterschieden werden.

In der DE 195 14 433 C1 ist eine Tracheostomiekanüle zum Einsatz in einem Tracheostoma beschrieben, welche eine schlauchförmige Außenkanüle aufweist, in welche eine ebenfalls schlauchförmige Innenkanüle führbar und mit der Außenkanüle am proximalen Teil verriegelbar ist, um ein Kanülenrohr zu bilden. Vorgesehen ist es, dass im proximalen Bereich der Außenkanüle ein Kanülenschild zur Anlage am Hals angebracht ist, durch welches hindurch der proximale Teil der Außenkanüle greift.

Die Aufgabe der Druckschrift besteht darin, eine gattungsgemäße Tracheostomiekanüle so weiterzubilden, dass die Verschwenkbarkeit um zwei Raumachsen im ausreichenden Maße möglich und technisch realisierbar wird.

Zur Lösung der Aufgabe wird vorgesehen, dass die Verschwenkbarkeit um die Y-Raumachse durch einen um die Außenkanüle greifenden und an dieser drehgelagerten Ring und die Verschwenkbarkeit um die X-Achse durch die Drehlagerung des Kanülenschildes an dem Ring erzielt wird. Betrachtet man das Kanülenschild idealisiert als Ring, so ist die Erfindung dadurch realisiert, dass zwei gegeneinander verdrehbare Ringe, nämlich der Innenring gegenüber dem Außenring (Kanülenschild), verschwenkbar an der Außenkanüle gelagert sind.

Diese Lösung ermöglicht nur das Schwenken einer Tracheostomiekanüle um eine X- und eine Y-Raumachse mittels einer mechanisch aufwendigen Anordnung. Kräfte, welche durch ein Verdrehen eines am Anschlussteil oder Konnektor angebrachten Schlauchs auf die Tracheostomiekanüle einwirken, werden durch die vorgeschlagene Lösung nicht abgebaut und führen zu einer Verschlechterung des Tragekomforts der Tracheostomiekanüle und möglicherweise auch zu Schmerzen beim Patienten.

Aus der DE 10 2005 030 300 ist eine Trachealkanüle mit einem beweglichen Schild bekannt.

Die zu lösende Aufgabe dieser Druckschrift besteht darin, eine Trachealkanüle mit beweglichem Schild anzugeben, die eine verbesserte Beweglichkeit ermöglicht und auch bei Dauerbelastung eine hohe mechanische Stabilität aufweist. Zur Lösung dieser Aufgabe wird vorgeschlagen, dass die Trachealkanüle aus einer rohr- oder schlauchförmigen Kanüle für die Hindurchführung durch einen Hals- und Luftröhrenschnitt in die Luftröhre eines Patienten, in welche eine ebenfalls schlauchförmige Innenkanüle führbar und mit der Kanüle verriegelbar ist, und einem an der Kanüle beweglich angebrachten Kanülenschild, durch welches hindurch die Kanüle greift, zur Anlage am Hals, durch dessen Anlage am Hals des Patienten die Position der Kanüle bzw. des Endes der Kanüle in der Luftröhre des Patienten definiert wird, besteht, wobei die Kanüle gegenüber dem Kanülenschild auf einer dem Patienten zugewandten Seite beweglich gelagert ist.

Nachteilig an dieser Lösung ist es, dass nur Bewegungen oder Lageunterschiede zwischen dem im Patienten angeordneten Teil der Trachealkanüle und dem am Hals des Patienten anliegenden Kanülenschild ausgeglichen werden. Bewegungen oder Verdrehungen, welche durch einen außerhalb des Patienten an der Trachealkanüle angeschlossenem Schlauch verursacht werde, können nicht ausgeglichen werden.

Nachfolgende Druckschrift WO 2015/162396 offenbart eine weitere bekannte Trachealkanüle mit Kugelgelenkkopf.

Die Aufgabe der Erfindung besteht nunmehr darin, eine Trachealkanüle mit einem Konnektor anzugeben, bei welcher der Konnektor mit einem gebogenen Rohr mittels einer einfach zu realisierenden, dreh- und schwenkbaren Verbindung angeordnet ist. Außerdem soll diese Verbindung für einen bestimmungsgemäßen Gebrauch der Trachealkanüle als eine unlösbare Verbindung ausgeführt sein.

Die Aufgabe wird durch eine Anordnung mit den Merkmalen gemäß Patentanspruch 1 der selbstständigen Patentansprüche gelöst. Weiterbildungen sind in den abhängigen Patentansprüchen 2 bis 8 angegeben.

Die Aufgabe wird auch durch eine Verfahren mit den Merkmalen gemäß Patentanspruch 9 der selbstständigen Patentansprüche gelöst. Eine Weiterbildung ist in dem abhängigen Patentanspruch 10 angegeben.

Vorgesehen ist es, ein Ende des Konnektors in der Form eines Gelenkkopfteils eines Kugelgelenks auszubilden. Dabei kann eine derartige Ausbildung einen Gelenkkopf eines Kugelgelenks auch nur teilweise ausbilden, solange die Funktionsfähigkeit des auszubildenden Kugelgelenks sichergestellt ist.

Zum Fixieren dieses Gelenkkopfteils wird eine aus zwei Teilen bestehende Gelenkpfanne bereitgestellt. Ein erster Teil dieser Gelenkpfanne oder die erste Teilgelenkpfanne wird an einem dem Konnektor zugewandten Ende des gebogenen Rohrs bereitgestellt. Der zweite Teil dieser Gelenkpfanne bzw. die zweite Teilgelenkpfanne wird durch eine Rastpfanne realisiert.

Derart ist es möglich, den Konnektor mit seinem Gelenkkopfteil in den beiden Teilen der Gelenkpfanne formschlüssig und beweglich anzuordnen. Zur Befestigung der Rastpfanne an dem beschriebenen ersten Ende des gebogenen Rohrs ist ein Verschluss vorgesehen. Gemäß dieser Ausführung ist der Konnektor einerseits in den Raumrichtungen X und Y schwenkbar und andererseits um seine Längsachse drehbar angeordnet. Durch diese freie Beweglichkeit des Konnektors können Kräfte, welche beispielsweise von einem am Konnektor angeschlossenem Beatmungsschlauch ausgehen, nicht auf den Patienten einwirken. Ihre negativen Auswirkungen, wie ein Druckgefühl oder Schmerzen bei einem Patienten, werden verringert.

Vorgesehen ist es, den Verschluss als einen Verschlussring mit Mitteln zum Einrasten zu versehen oder den Verschluss als einen Bajonettverschluss auszuführen. In einer Ausführung weist dieser Verschlussring an einem ersten Ende Mittel zum Einrasten, wie beispielsweise eine Wulst und eine Nut, und an einem zweiten Ende einen Bajonettverschluss auf. Ein derartiger Verschlussring ermöglicht somit eine einfache Montage und Demontage der Trachealkanüle.

Beispielsweise kann das erste Ende des Verschlussrings mittels seiner Wulst in eine Nut der Rastpfanne einrasten, während eine Verbindung des Verschlussrings an seinem zweiten Ende mit dem Außenrohr über einen Bajonettverschluss erfolgt.

Vorgesehen ist es weiterhin, den Konnektor, den Verschlussring und die Rastpfanne aus einem harten Kunststoff, wie beispielsweise Acrylnitril-Butadien-Styrol (ABS), zu fertigen. Die Verwendung eines derartigen Kunststoffs ermöglicht eine robuste, funktional hochwertige Ausführung der beweglichen Verbindung zwischen dem gebogenen Rohr und dem Konnektor. Hierbei wird unter einem derartigen, harten Kunststoff ein Werkstoff mit einer Shore-Härte im Bereich von 50 Shore-D bis 90 Shore-D, bevorzugt im Bereich zwischen 65 Shore-D bis 75 Shore-D, verstanden. Für den Konnektor, den Verschlussring und die Rastpfanne können gleiche oder verschiedene Werkstoffe zum Einsatz kommen, welche die angegebenen Bedingungen an die Shore-Härte erfüllen.

Weiterhin vorgesehen ist es auch, das gebogene Rohr aus einem weichen flexiblen Kunststoff, wie beispielsweise Polyurethan oder EVA (Ethylen Vinylacetat) auszuführen. Als ein derartiger weicher Kunststoff wird ein Werkstoff mit einer Shore-Härte im Bereich von 40 Shore-A bis 90 Shore-A, bevorzugt 60 Schore-A bis 70 Shore-A, verstanden.

Das gebogene Rohr weist außerdem einen von seiner Längsachse abweichenden gebogenen Verlauf auf, welcher einem üblichen gebogenen Außenrohr angepasst ist.

Die zuvor erläuterten Merkmale und Vorteile dieser Erfindung sind nach sorgfältigem Studium der nachfolgenden ausführlichen Beschreibung der hier bevorzugten, nicht einschränkenden Beispielausgestaltungen der Erfindung mit den zugehörigen Zeichnungen besser zu verstehen und zu bewerten, welche zeigen:
- Fig. 1:: eine Ausführungsform der erfindungsgemäßen Trachealkanüle in einer Prinzipdarstellung,
- Fig. 2:: eine Draufsicht und eine Schnittdarstellung der Ausführungsform nach Figur 1,
- Fig. 3:: eine auszugsweise vergrößerte Schnittdarstellung der Verbindung des gebogenen Rohrs mit dem Konnektor nach Figur 2,
- Fig. 4:: ein Beispiel der Kopplung der erfindungsgemäßen Anordnung mit einem Außenrohr und
- Fig. 5:: eine perspektivische Darstellung der erfindungsgemäßen Trachealkanüle.

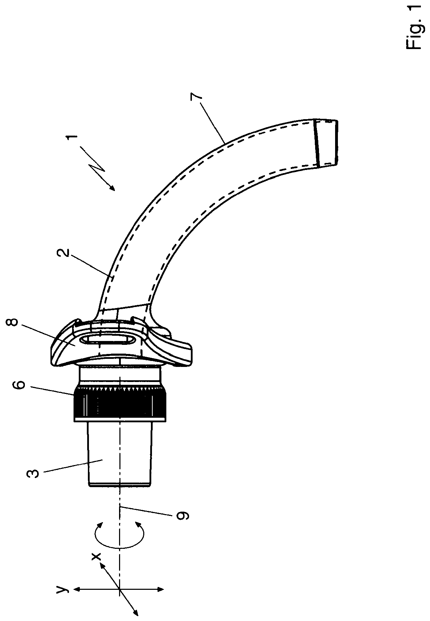

Die Figur 1 zeigt eine Ausführungsform der erfindungsgemäßen Trachealkanüle 1 in einer nicht maßstabsgetreuen Prinzipdarstellung. Die Trachealkanüle 1 umfasst ein gebogenes Rohr 2, welches mittels einer Strich-Strich-Linie dargestellt ist. Dieses gebogene Rohr 2 kann beispielsweise in einem Außenrohr 7 angeordnet werden, welches durch einen Hals- und Luftröhrenschnitt hindurch bis in die Luftröhre des Patienten hinein angeordnet ist. In dieser Position der Trachealkanüle 1 liegt das Kanülenschild 8 am Hals eines Patienten an. Eine Anordnung des gebogenen Rohrs 2 in einem Außenrohr 7 ist eine Option und für die vorliegende Erfindung nicht zwingend erforderlich.

Die Trachealkanüle 1 umfasst weiterhin einen Verschluss, der als ein Verschlussring 6 ausgebildet ist, welcher das gebogene Rohr 2 mit dem Konnektor 3 formschlüssig derart verbindet, dass der Konnektor 3 sowohl in den gezeigten Raumrichtungen X und Y schwenkbar als auch um seine Längsachse 9 drehbar gelagert mit dem gebogenen Rohr 2 verbunden ist. Eine derartige Schwenkbewegung des Konnektors 3, abweichend von seiner mittigen Ausrichtung, ist in der Figur 1 nicht dargestellt. Der Konnektor 3 ist an einem von einem Patienten abgewandten Ende einer Trachealkanüle 1 angeordnet. Dieses Ende wird alternativ auch als maschinenseitiges Ende bezeichnet.

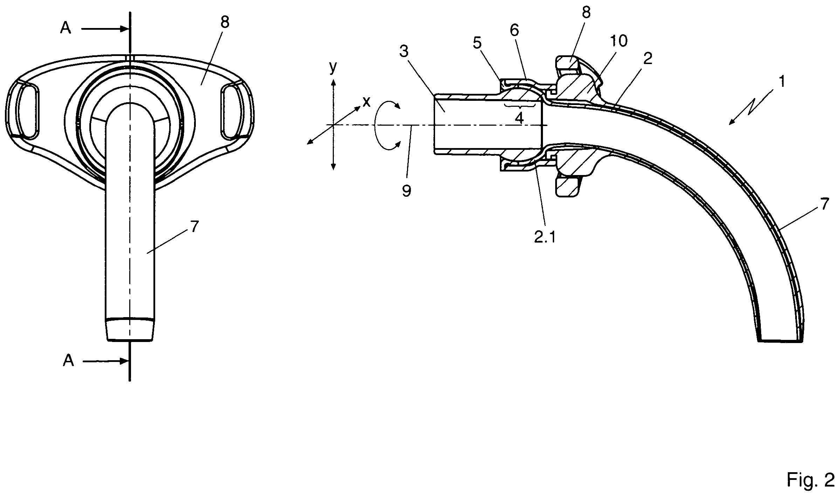

In der Figur 2 ist im linken Teil der Figur eine Sicht auf die Trachealkanüle 1 entlang der Längsachse 9 und im rechten Teil eine Schnittdarstellung entlang der dargestellten Schnittlinie AA gezeigt.

Die Darstellung im linken Teil der Figur 2 ermöglicht somit eine Sicht auf das gebogene Außenrohr 7 sowie das Kanülenschild 8.

Der rechte Teil der Figur 2 zeigt in der Schnittdarstellung einen Konnektor 3, welcher beispielsweise ein üblicher Standard-Konnektor 3 mit einem Durchmesser von 15 Millimetern sein kann. Dieser Konnektor 3 ist an seinem dem gebogenen Rohr 2 zugewandten ersten Ende in der Form eines sogenannten Gelenkkopfes eines Kugelgelenks ausgeführt. Dabei wird durch dieses erste Ende des Konnektors 3 nur ein Teil eines kugelähnlichen Gelenkkopfes eines bekannten Kugelgelenks, ein sogenannter Gelenkkopfteil 4, wie im rechten Teil der Figur 2 gezeigt, ausgebildet. Der Konnektor 3 weist an diesem ersten Ende auch weiterhin eine zum gebogenen Rohr 2 ausgerichtete Öffnung auf.

Zur Fixierung des Gelenkkopfes eines Kugelgelenks ist ein Gelenkkopf nach dem bekannten Stand der Technik in einer sogenannten Gelenkpfanne angeordnet. Diese den Gelenkkopf zumindest teilweise umschließende Gelenkpfanne ist in der vorliegenden Erfindung durch zwei Teile oder zwei Teilgelenkpfannen 2.1 und 5 realisiert. Derart wird eine leichte Montage oder Demontage, also ein Herstellen der Verbindung des gebogenen Rohrs 2 mit dem Konnektor 3 sowie ein Lösen dieser Verbindung, ermöglicht und erleichtert.

Eine erste Teilgelenkpfanne 2.1 wird durch das erste dem Konnektor 3 zugewandte Ende des gebogenen Rohrs 2 ausgebildet. In diese erste Teilgelenkpfanne 2.1 wird das erste Ende des Konnektors 3, also der Gelenkkopfteil 4, eingelegt bzw. angeordnet. Zur weiteren Ausformung der Gelenkpfanne ist eine Rastpfanne 5 angeordnet, welche die zweite Teilgelenkpfanne ausbildet und eine formschlüssige Aufnahme des Gelenkkopfteils 4 des Konnektors 3 ermöglicht.

Zum Fixieren des am ersten Endes des Konnektors 3 angeordneten Gelenckopfteils 4 mit der ersten Teilgelenkpfanne 2.1 und der Rastpfanne 5 ist ein Verschlussring 6 vorgesehen. Der Verschlussring 6 kann beispielsweise mit dem Außenrohr 7 in der Form eines Bajonettverschlusses verbunden sein. Dieser Verschlussring 6 kann beispielsweise über das gebogene Rohr 2 sowie über die erste Teilgelenkpfanne 2.1 und die Rastpfanne 5 geschoben werden und in der im rechten Teil der Figur 2 dargestellten Position an einem an der Rastpfanne 5 angeordneten, geeigneten Mittel einrasten. Alternativ kann der Verschlussring 6 die Verbindung zur Rastpfanne 5 auch in der Form eines Bajonettverschlusses herstellen.

Das Fixieren der Rastpfanne 5 in der gezeigten Position an der ersten Teilgelenkpfanne 2.1 und die Aufnahme des teilweise als Kugelgelenk ausgebildeten Gelenkkopfteils 4 des Konnektors 3 in der beschriebenen Weise stellt eine formschlüssige und durch den Verschlussring 6 leicht lösbare Verbindung zwischen dem gebogenen Rohr 2 und dem Konnektor 3 bereit. Dieser Formschluss ermöglicht es, dass sich der Konnektor 3 sowohl in den Raumrichtungen X und Y bewegen kann als auch um seine Längsachse 9 drehbar ist. Kräfte, welche beispielsweise von einem mit dem Konnektor 3 verbundenen Beatmungsschlauch auf den Konnektor 3 der Trachealkanüle 1 ausgeübt werden, können durch diese Bewegungsfreiheit des Konnektors 3 vom Patienten ferngehalten werden. Somit wird der Tragkomfort der Trachealkanüle 1 verbessert und eine Belastung des Patienten verringert. Außerdem wird die Gefahr eines Lösens des Beatmungsschlauches vom Konnektor 3 durch die Beweglichkeit des Konnektors 3 verringert.

Vorgesehen ist es, den Konnektor 3, den Verschlussring 6 sowie die Rastpfanne 5 aus einem harten, gut zu verarbeitenden und formstabilen Kunststoff herzustellen, während das gebogene Rohr 2 vorzugsweise aus einem weichen Kunststoff gefertigt wird. Als harter Kunststoff kann beispielsweise Acrylnitril-Butadien-Styrol-Copolymer (ABS) zum Einsatz kommen.

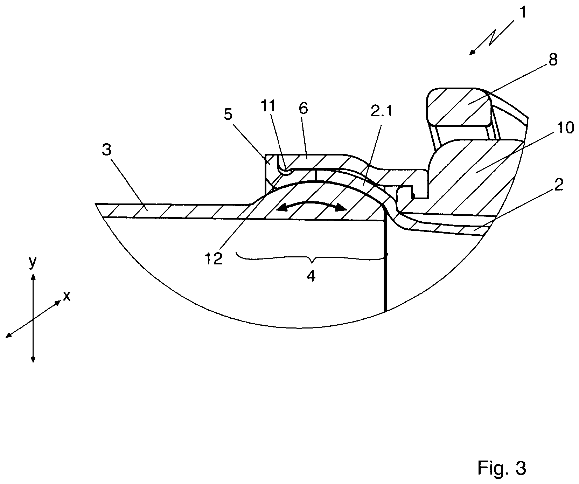

In der Figur 3 ist ein Teil der Verbindung des gebogenen Rohrs 2 mit dem Konnektor 3 auszugsweise vergrößert in einer Schnittdarstellung gemäß dem rechten Teil der Figur 2 gezeigt. Dargestellt ist das erste Ende des Konnektors 3, welches den Gelenkkopfteil 4 ausbildet. Wie zu erkennen ist, ist dieser Gelenkkopfteil 4 an das erste Ende des gebogenen Rohrs 2 angelegt, welches die erste Teilgelenkpfanne 2.1 ausbildet. Die zweite Teilgelenkpfanne wird durch die Rastpfanne 5 gebildet. Diese Gelenkpfanne, gebildet durch die erste und die zweite Teilgelenkpfanne, ist derart dimensioniert, dass der Gelenkkopfteil 4 des Konnektors 3 weit genug umschlossen wird, um eine formschlüssige, den Gelenkkopfteil 4 sicher haltende Verbindung herzustellen. Andererseits wird der Gelenkkopfteil 4 nicht vollständig durch die ausgebildete Gelenkpfanne umschlossen, um die Beweglichkeit des Konnektors 3 in den Raumrichtungen X und Y zu ermöglichen.

Als Beispiel ist in der Figur 3 mit dem im Gelenkkopfteil 4 eingezeichneten Doppelpfeil die Beweglichkeit des Konnektors 3 in der Raumrichtung Y dargestellt. Auf die Darstellung der ebenfalls vorhandenen Beweglichkeit in der Raumrichtung X wurde in der Figur 3 ebenso wie auf die Darstellung der Beweglichkeit um die nicht dargestellte Längsachse 9 des Konnektors 3, verzichtet, da dies dem Fachmann bei Kugelgelenken allgemein bekannt ist.

Die Figur 3 zeigt den über das erste Ende des gebogenen Rohrs 2 geschobenen und mit der Rastpfanne 5 verbundenen Verschlussring 6, welcher nach einem Einrasten in der Rastpfanne 5 das gebogene Rohr 2, die Rastpfanne 5 und den Konnektor 3 sicher zusammenhält.

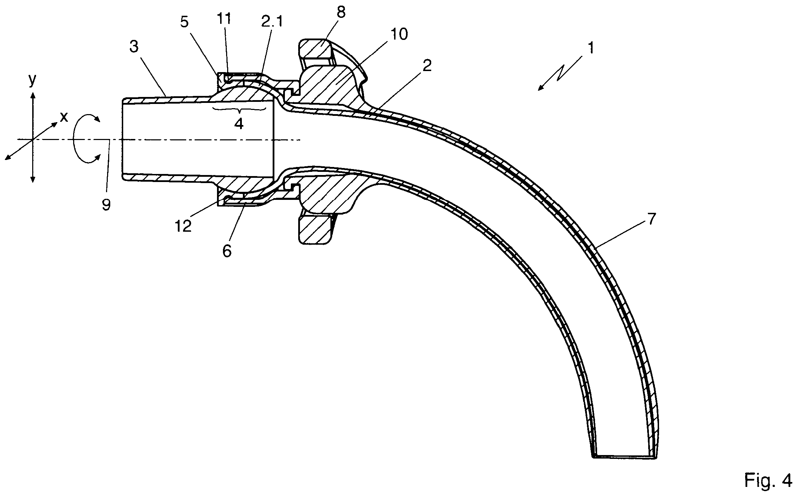

Die Figur 4 zeigt das aus dem gebogenen Rohr 2 mit der ersten Teilgelenkpfanne 2.1, der Rastpfanne 5, dem Verschlussring 6 und dem Konnektor 3 bestehende Teilsystem in einer Verbindung mit einem Außenrohr 7.

Vorgesehen ist es, dieses Teilsystem mit einem Außenrohr 7 zu verbinden und das System derart zu einer einsatzbereiten Trachealkanüle 1 zu komplettieren, wobei das Außenrohr 7 in einem Hals- und Luftröhrenschnitt eines Patienten angeordnet ist und sich bis in die Luftröhre des Patienten hinein erstreckt.

Zu diesem Zweck ist das Außenrohr 7 beispielsweise mit einem Bereich eines zunehmenden Querschnitts 10 versehen, in welchem das Außenrohr 7 mit dem Kanülenschild 8 verbunden ist. Auf eine detaillierte Darstellung dieser Verbindung wurde verzichtet, da diese nach dem üblichen Stand der Technik ausgeführt sein kann. In dieser vervollständigten Ausführung ist die Trachealkanüle 1 zum üblichen Einsatz bei einem Patienten vorgesehen, wobei das Kanülenschild 8 bei eingebrachter Trachealkanüle 1 am Hals des Patienten anliegt.

In einer Ausführung ist der Verschlussring 6 derart ausgeführt, dass er mit dem Außenrohr 7 über einen ausgebildeten Bajonettverschluss verbindbar ist. Derart kann der Verschlussring 6 beispielsweise über den den Bajonettverschlussteil am Außenrohr 7 bildenden Teil geschoben und durch ein Verdrehen in üblicher Art fixiert werden. Das diesem Bajonettverschluss abgewandte Ende des Verschlussrings 6 kann derart ausgeführt sein, dass es eine zumindest teilweise umlaufende, den Durchmesser des Verschlussrings 6 reduzierende Wulst 11 aufweist. In diesem Fall wird die Rastpfanne 5 derart ausgebildet, dass diese eine zumindest teilweise umlaufende, die Wulst 11 aufnehmende Nut 12 aufweist. Die Verbindung des Verschlussrings 6 mit der Rastpfanne 5 wird in diesem Fall durch ein Einrasten der Wulst 11 des Verschlussrings 6 in der Nut 12 der Rastpfanne 5 hergestellt. Eine verbesserte Darstellung der Elemente Wulst 11 und Nut 12 kann der Figur 3 entnommen werden.

Alternativ kann die Verbindung des Verschlussrings 6 mit der Rastpfanne 5 auch in der Art eines Bajonettverschlussringes ausgeführt werden.

Mittel, welche zur praktischen Ausführung eines Bajonettverschlussrings notwendig oder vorteilhaft sind, können von einem Fachmann in geeigneter Weise ergänzt werden.

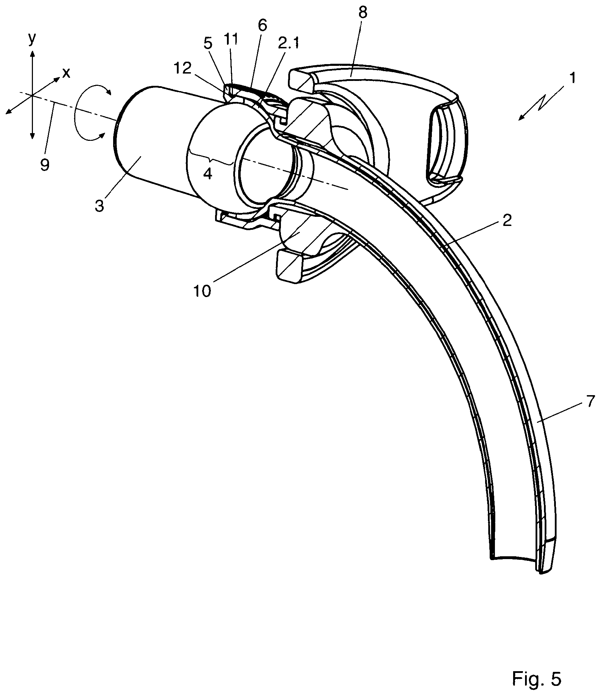

In der Figur 5 ist die Trachealkanüle 1 in einer perspektivischen Ansicht dargestellt, wobei der Konnektor 3 mit seinem ausgebildeten Gelenkkopfteil 4 im Gegensatz zu allen anderen Elementen nicht in einer Schnittdarstellung gezeigt ist. Die Darstellung der Figur 5 zeigt die aus zwei Teilen, also der ersten Teilgelenkpfanne 2.1 und der Rastpfanne 5, bestehende Gelenkpfanne, in welcher der Gelenkkopfteil 4 beweglich gelagert aufgenommen und fixiert wird, besonders anschaulich. Diese Befestigung des Gelenkkopfteils 4 ermöglicht die bereits beschriebene Schwenkbarkeit des Konnektors 3 in den Raumrichtungen X und Y und gleichzeitig seine Drehbarkeit um die Längsachse 9. Das im Außenrohr 7 angeordnete gebogene Rohr 2 kann in diesem Fall der Anordnung auch als Innenrohr einer Trachealkanüle 1 bezeichnet werden.

### Liste der Bezugszeichen

- 1: Trachealkanüle
- 2: gebogenes Rohr
- 2.1: erste Teilgelenkpfanne
- 3: Konnektor
- 4: Gelenkkopfteil
- 5: Rastpfanne (zweite Teilgelenkpfanne)
- 6: Verschlussring
- 7: Außenrohr
- 8: Kanülenschild
- 9: Längsachse
- 10: Bereich eines zunehmenden Querschnitts
- 11: Wulst
- 12: Nut

## Patentansprüche

1. Trachealkanüle (1), welche mindestens ein gebogenes Rohr (2) und einen Konnektor (3) aufweist, **dadurch gekennzeichnet, dass** an einem dem gebogenen Rohr (2) zugewandtem ersten Ende des Konnektors (3) ein Gelenkkopfteil (4) eines Kugelgelenks angeordnet ist, dass an einem dem Konnektor (3) zugewandtem ersten Ende des gebogenen Rohrs (2) eine erste Teilgelenkpfanne (2.1) des Kugelgelenks angeordnet ist, dass als eine zweite Teilgelenkpfanne des Kugelgelenks eine Rastpfanne (5) angeordnet ist und dass zur Verbindung der Rastpfanne (5) mit dem gebogenen Rohr (2) ein Verschlussring (6) angeordnet ist.

2. Trachealkanüle (1) nach Anspruch 1, **dadurch gekennzeichnet, dass** an einem ersten Ende des Verschlussrings (6) und an der Rastpfanne (5) erste Mittel zum Einrasten des Verschlussrings (6) in die Rastpfanne (5) angeordnet sind.

3. Trachealkanüle (1) nach Anspruch 2, **dadurch gekennzeichnet, dass** als erstes Mittel zum Einrasten des Verschlussrings (6) in die Rastpfanne (5) eine Wulst 11 und eine Nut 12 angeordnet sind.

4. Trachealkanüle (1) nach einem der Ansprüche 1 bis 3, **dadurch gekennzeichnet, dass** ein das gebogene Rohr (2) umgebendes Außenrohr (7) angeordnet ist und dass an einem zweiten Ende des Verschlussrings (6) und am Außenrohr (7) zweite Mittel zum Einrasten angeordnet sind.

5. Trachealkanüle (1) nach Anspruch 4, **dadurch gekennzeichnet, dass** das zweite Mittel zum Einrasten des Verschlussrings (6) ein Bajonettverschluss ist.

6. Trachealkanüle (1) nach einem der Ansprüche 1 bis 5, **dadurch gekennzeichnet, dass** das gebogene Rohr (2) aus einem weichen Kunststoff mit einer Shore-Härte von 40 Shore-A bis 90 Shore-A, bevorzugt 60 Schore-A bis 70 Shore-A, besteht und eine Biegung aufweist.

7. Trachealkanüle (1) nach einem der Ansprüche 1 bis 6, **dadurch gekennzeichnet, dass** der Konnektor (3), der Verschlussring (6) und die Rastpfanne (5) aus Acrylnitril-Butadien-Styrol gefertigt werden.

8. Trachealkanüle (1) nach einem der Ansprüche 1 bis 7, **dadurch gekennzeichnet, dass** der Konnektor (3) an einem zweiten Ende einen Durchmesser von 15 mm aufweist.

9. Verfahren zur Herstellung einer Trachealkanüle (1), bei welchem ein gebogenes Rohr (2) mit einem Konnektor (3) verbunden wird, **dadurch gekennzeichnet, dass** ein Konnektor (3) mit einem Gelenkkopfteil (4) bereitgestellt wird, dass an einem dem Konnektor (3) zugewandtem ersten Ende des gebogenen Rohrs (2) eine erste Teilgelenkpfanne (2.1) am gebogenen Rohr (2) bereitgestellt wird, dass eine Rastpfanne (5) als eine zweite Teilgelenkpfanne bereitgestellt wird, dass der Gelenkkopfteil (4) des Konnektors (3) durch die beiden Teilgelenkpfannen (2.1 und 5) formschlüssig und bewegbar fixiert wird, dass eine Arretierung der beiden Teilgelenkpfannen (2.1 und 5) mittels eines Verschlussrings (6) erfolgt, wobei der Konnektor (3) mit seinem Gelenkkopfteil (4) in Raumrichtungen X und Y schwenkbar und um seine Längsachse (9) drehbar fixiert ist.

10. Verfahren nach Anspruch 9, **dadurch gekennzeichnet, dass** der Verschlussring (6) an einem ersten Ende mit einem Mittel zum Einrasten bereitgestellt wird und dass der Verschlussring (6) an einem zweiten Ende mit einem Bajonettverschluss bereitgestellt wird.

## Claims

1. Tracheal cannula (1) having at least one curved tube (2) and a connector (3), **characterized in that** a joint head part (4) of a ball-and-socket joint is arranged at a first end of the connector (3) facing towards the curved tube (2), **in that** a first partial joint socket (2.1) of the ball-and-socket joint is arranged at a first end of the curved tube (2) facing towards the connector (3), **in that** a latching socket (5) is arranged as a second partial joint socket of the ball-and-socket joint, and **in that** a closure ring (6) is arranged for connecting the latching socket (5) to the curved tube (2).

2. Tracheal cannula (1) according to Claim 1, **characterized in that** first means for latching the closure ring (6) into the latching socket (5) are arranged at a first end of the closure ring (6) and on the latching socket (5).

3. Tracheal cannula (1) according to Claim 2, **characterized in that** a bead 11 and a groove 12 are arranged as first means for latching the closure ring (6) into the latching socket (5).

4. Tracheal cannula (1) according to one of Claims 1 to 3, **characterized in that** an outer tube (7) is arranged surrounding the curved tube (2), and **in that** second means for latching are arranged at a second end of the closure ring (6) and on the outer tube (7).

5. Tracheal cannula (1) according to Claim 4, **characterized in that** the second means for latching of the closure ring (6) is a bayonet catch.

6. Tracheal cannula (1) according to one of Claims 1 to 5, **characterized in that** the curved tube (2) is made of a soft plastic, with a Shore hardness of 40 Shore A to 90 Shore A, preferably 60 Shore A to 70 Shore A, and has a bend.

7. Tracheal cannula (1) according to one of Claims 1 to 6, **characterized in that** the connector (3), the closure ring (6) and the latching socket (5) are manufactured from acrylonitrile-butadiene-styrene.

8. Tracheal cannula (1) according to one of Claims 1 to 7, **characterized in that** the connector (3) has a diameter of 15 mm at a second end.

9. Method for producing a tracheal cannula (1), in which method a curved tube (2) is connected to a connector (3), **characterized in that** a connector (3) with a joint head part (4) is made available, **in that** a first partial joint socket (2.1) on the curved tube (2) is made available at a first end of the curved tube (2) facing towards the connector (3), **in that** a latching socket (5) is made available as a second partial joint socket, **in that** the joint head part (4) of the connector (3) is positively and movably fixed by the two partial joint sockets (2.1 and 5), **in that** a locking of the two partial joint sockets (2.1 and 5) takes place by means of a closure ring (6), wherein the connector (3) with its joint head part (4) is fixed pivotably in spatial directions X and Y and rotatably about its longitudinal axis (9).

10. Method according to Claim 9, **characterized in that** the closure ring (6) is provided at a first end with a means for latching, and **in that** the closure ring (6) is provided at a second end with a bayonet catch.

## Revendications

1. Canule trachéale (1), qui présente au moins un tube courbe (2) et un connecteur (3),
**caractérisée en ce qu'**une partie de tête d'articulation (4) d'une articulation sphérique est disposée à une première extrémité du connecteur (3), tournée vers le tube courbe (2), **en ce qu'**un premier coussinet d'articulation partiel (2.1) de l'articulation sphérique est disposé à une première extrémité du tube courbe (2), tournée vers le connecteur (3), **en ce qu'**un coussinet d'encliquetage (5) est disposé comme deuxième coussinet d'articulation partiel de l'articulation sphérique, et **en ce qu'**une bague de fermeture (6) est disposée pour relier le coussinet d'encliquetage (5) au tube courbe (2).

2. Canule trachéale (1) selon la revendication 1, **caractérisée en ce que** des premiers moyens pour l'encliquetage de la bague de fermeture (6) dans le coussinet d'encliquetage (5) sont disposés à une première extrémité de la bague de fermeture (6) et sur le coussinet d'encliquetage (5).

3. Canule trachéale (1) selon la revendication 2, **caractérisée en ce qu'**un bourrelet (11) et une rainure (12) sont disposés comme premiers moyens pour l'encliquetage de la bague de fermeture (6) dans le coussinet d'encliquetage (5).

4. Canule trachéale (1) selon l'une quelconque des revendications 1 à 3, **caractérisée en ce qu'**un tube extérieur (7) entourant le tube courbe (2) est disposé, et **en ce que** des deuxièmes moyens pour l'encliquetage sont disposés à une deuxième extrémité de la bague de fermeture (6) et sur le tube extérieur (7).

5. Canule trachéale (1) selon la revendication 4, **caractérisée en ce que** le deuxième moyen pour l'encliquetage de la bague de fermeture (6) est une fermeture à baïonnette.

6. Canule trachéale (1) selon l'une quelconque des revendications 1 à 5, **caractérisée en ce que** le tube courbe (2) est composé d'une matière plastique souple d'une dureté Shore de 40 Shore-A à 90 Shore-A, de préférence de 60 Shore-A à 70 Shore-A, et présente une courbure.

7. Canule trachéale (1) selon l'une quelconque des revendications 1 à 6, **caractérisée en ce que** le connecteur (3), la bague de fermeture (6) et le coussinet d'encliquetage (5) sont fabriqués en acrylonitrile butadiène styrène.

8. Canule trachéale (1) selon l'une quelconque des revendications 1 à 7, **caractérisée en ce que** le connecteur (3) présente à une deuxième extrémité un diamètre de 15 mm.

9. Procédé de fabrication d'une canule trachéale (1), dans lequel un tube courbe (2) est relié à un connecteur (3), **caractérisé en ce qu'**un connecteur (3) doté d'une partie de tête d'articulation (4) est prévu, **en ce qu'**un premier coussinet d'articulation partiel (2.1) est prévu sur le tube courbe (2) à une première extrémité du tube courbe (2), tournée vers le connecteur (3), **en ce qu'**un coussinet d'encliquetage (5) est prévu comme deuxième coussinet d'articulation partiel, **en ce que** la partie de tête d'articulation (4) du connecteur (3) est fixée par complémentarité de forme et de manière mobile par les deux coussinets d'articulation partiels (2.1 et 5), **en ce que** les deux coussinets d'articulation partiels (2.1 et 5) sont arrêtés au moyen d'une bague de fermeture (6), le connecteur (3) doté de sa partie de tête d'articulation (4) étant fixé de manière à pouvoir pivoter dans les directions spatiales X et Y et tourner autour de son axe longitudinal (9).

10. Procédé selon la revendication 9, **caractérisée en ce que** la bague de fermeture (6) est munie d'un moyen d'encliquetage à une première extrémité, et **en ce que** la bague de fermeture (6) est munie d'une fermeture à baïonnette à une deuxième extrémité.
